# EUROPEAN PATENT SPECIFICATION

(11) **EP 1 859 725 B1**
(45) Date of publication and mention of the grant of the patent: **05.12.2012**
(21) Application number: 06729060.1
(22) Date of filing: 14.03.2006
(51) Int. Cl.: A61B 1/005, B29C 73/02, G02B 23/24, B29C 61/02, A61B 1/00

(54) **ENDOSCOPE AND REPAIR METHOD OF ENDOSCOPE**
ENDOSKOP UND VERFAHREN ZUR REPARATUR EINES ENDOSKOPS
ENDOSCOPE ET PROCEDE DE REPARATION

(30) Priority: 14.03.2005 JP 2005071203
(43) Date of publication of application: 28.11.2007
(73) Proprietor: Olympus Corporation, Tokyo 151-0072 (JP)
(72) Inventor: NAKAMURA, Takeaki c/o Olympus Intel.Property Serv.Co.,Ltd, Hachioji-shi Tokyo 192-8512 (JP)
(74) Representative: von Hellfeld, Axel
(86) International application number: PCT/JP2006/305026
(87) International publication number: WO 2006/098322

(56) References cited:
- EP-A- 0 430 542
- JP-A- 2000 166 859
- JP-A- 2000 320 785
- JP-A- 2002 125 916
- US-A- 6 083 152

## Description

### Technical Field

The present invention relates to an endoscope for dealing with a case that a site to be repaired has occurred on an outer skin configuring an outer surface of a flexible pipe according to use thereof, and a repair method of an endoscope.

### Background Art

For example, as shown in Jpn. Pat. Appln. KOKAI Publication No. 01-227735 (Patent Document 1), an endoscope used in a medical field includes a grasping section that is grasped by one hand of an operator and an operation section that is provided with knobs to be operated by the other hand of the operator, a forceps channel, or the like. A proximal end portion of an insertion section inserted into a body cavity of a patient is coupled to this operation section.

The insertion section includes a soft section, a bending section, and a distal end hard section. The distal end hard section is provided with an air-supplying/water-supplying nozzle and lenses (an objective lens system). Since the soft section and the bending portion have flexibility, they are generally collectively called "flexible pipe".

The operator inserts the insertion section into a desired site in a body cavity of a patient. The operator operates the operation section to bend the bending section. Thereby, the operator can cause the distal end hard section to face an affected area and capture an image of the desired site. The operator can confirm the captured image to perform proper examination/procedure to the affected area. The endoscope that has been used for conducting required works is cleaned, disinfected, and subjected to sterilization treatment.

JP 2000-166859 discloses an endoscope, by which a section of the outer periphery of a tip end constituting member, which constitutes a tip end section is covered with the tip end section of a covering tube, which is the shell of a curved section and is provided with a thread from above. An adhesive is applied to the thread and covered by a covering tube.

EP 0 430 542 A2 discloses a method of producing a flexible tube. A braid is fitted on an outer periphery of an elongated core member of a cylindrical shape. Then, a soft resin layer is formed on an outer periphery of the braid, and the resin layer and the braid are joined together to form a sheath. The core member is removed from the sheath. Subsequently, a flex in form of a spirally-wound metal strip is inserted into the sheath. The core member can be removed from the sheath by dipping the core member in a dissolvable solution.

US 6,083,152 discloses an endoscopic insertion tube including a helically wound spiral tube and a net-like braid disposed in overlaying relation. A polymeric adhesive layer is applied over the length of the spiral tube and the outer peripheral surface of the braid. A pre-expanded hollow tubular sheath member is placed over the adhesive layer and is shrunk thereupon using heat to cross-link with the polymeric adhesive layer.

The invention is defined by the appended claims.

Using such an endoscope, examination/procedure, and cleaning/disinfecting/sterilizing treatment are generally conducted repeatedly for a prolonged period. Therefore, it is inevitable that an outer skin configuring an outer surface of the insertion section is worn due to the long-term use. Ultimately, a hole, damage or the like may occur in the outer skin. When these phenomena are confirmed or when there are indications that the phenomena occur, a whole outer skin must be replaced with a new one.

Especially, the insertion section of the endoscope is put in such a use environment that it is inserted into a body cavity of a patient. Therefore, it is required to conduct a replacing work with a considerably high quality. Specifically, a treatment for peeling off a whole outer skin, a treatment for bonding a new outer skin, a treatment for coating a top coat layer on a surface of the outer skin after bonded, and the like must be performed while maintaining high quality. Further, since these treatment works are performed by use of a heating furnace, it is not only required to provide major equipment but also much labor and time are required.

In view of these circumstances, the present invention has been made, and an object thereof is to provide an endoscope where a site to be repaired occurring on an outer skin of a flexible pipe according to use of the endoscope can be repaired relatively easily in a short time so that improvement of workability can be achieved and that can withstand long-term use without impairing mechanical strength of the flexible pipe and a repair method of an endoscope.

An endoscope in one aspect of the present invention comprises: adhesive that is applied to a site to be repaired which has occurred on an outer surface of a flexible pipe inserted into a body cavity according to use of the endoscope; and a thermally shrinkable tube that is fitted on the site to be repaired applied with the adhesive so as to face the site to be repaired and is tightly attached to an outer surface of the flexible pipe via the adhesive according to shrinking of the thermally shrinkable tube due to heating thereof.

By applying adhesive to a site to be repaired and fitting thermally-shrinkable tube thereon to conduct heating shrinkage to the tube in this manner, repair can be performed relatively easily in a short time so that workability can be improved and an endoscope that can withstand long-term use without impairing mechanical strength of a flexible pipe can be obtained.

An endoscope in another aspect of the present invention comprises: a thermally shrinkable tube that is fitted on a site to be repaired which has occurred on an outer surface of a flexible pipe inserted into a body cavity according to use of the endoscope so as to face the site to be repaired; and an adhesive layer that is provided on an inner peripheral face of the thermally shrinkable tube and tightly attaches the thermally shrinkable tube on an outer surface of the flexible pipe according to shrinking of the thermally shrinkable tube due to heating thereof.

By fitting a thermally-shrinkable tube whose inner face is provided with an adhesive layer on a site to be repaired to conduct heating shrinkage of the tube in this manner, repair can be performed relatively easily in a short time so that workability can be improved and an endoscope that can withstand long-term use without impairing mechanical strength of a flexible pipe can be obtained.

An endoscope that is provided with an insertion section to be inserted into a body cavity comprising a flexible pipe including a bending section and a soft section, and a distal end hard section provided at a distal end of the flexible pipe, in another aspect of the present invention comprises: a new outer skin that configures an outer surface of the bending section and by which an outer skin where a site to be repaired has occurred according to use of the endoscope has been replaced; a first string-winding section that fixedly couples the bending section and the distal end hard section by winding a string from a distal end of the newly replaced outer skin to a rear end of the distal end hard section; a second string-winding section that fixedly couples the bending section and the soft section by winding a string from a proximal end of the newly replaced outer skin to a distal end of the soft section; adhesives that are respectively applied to the first string-winding section and the second string-winding section; and thermally shrinkable tubes that are respectively fitted on the first string-winding section and the second string-winding section applied with the adhesives through clearances, while being respectively opposed thereto, and are respectively tightly attached to the first string-winding section and the second string-winding section via the adhesives according to shrinking thereof due to heating.

By replacing only an outer skin of the bending section where a site to be repaired has occurred by a new outer skin, fixedly coupling a distal end and a proximal end of the new outer skin by utilizing a string winding portion, and applying adhesive to the string-winding portion and fitting a thermally-shrinkable tube thereon to conduct heating shrinkage of the tube in this manner, repair can be performed relatively easily in a short amount of time so that workability can be improved and an endoscope that can withstand long-term use without impairing mechanical strength of a flexible pipe can be obtained.

A repair method of an endoscope for repairing a site to be repaired which has occurred on an outer skin configuring an outer surface of a flexible pipe according to use of the endoscope, in another aspect of the present invention comprises: a first step of applying adhesive to the site to be repaired on the outer skin; a second step of fitting a thermally shrinkable tube on the site to be repaired on the outer skin applied with the adhesive; and a third step of heating and shrinking the thermally shrinkable tube to tightly attach the thermally shrinkable tube on the outer skin by the adhesive.

By applying adhesive to a site to be repaired and fitting a thermally-shrinkable tube on the site to be repaired from above the adhesive to conduct heating shrinkage in this manner, repair can be performed relatively easily in a short time so that workability can be improved and an endoscope that can withstand long-term use without impairing mechanical strength of a flexible pipe can be obtained.

A repair method of an endoscope for repairing a site to be repaired which has occurred on an outer skin configuring an outer surface of a flexible pipe according to use of the endoscope, comprises: a first step of fitting a thermally shrinkable tube having an adhesive layer on an inner peripheral face thereof on the site to be repaired on the outer skin; and a second step of heating and shrinking the thermally shrinkable tube to tightly attach the thermally shrinkable tube to the outer skin via the adhesive layer.

By fitting a thermally-shrinkable tube whose inner face is provided with an adhesive layer on a site to be repaired to conduct heating shrinkage in this manner, repair can be performed relatively easily in a short time so that workability can be improved and an endoscope that can withstand long-term use without impairing mechanical strength of a flexible pipe can be obtained.

A repair method of an endoscope that is provided with an insertion section to be inserted into a body cavity comprising a flexible pipe including a bending section and a soft section, and a distal end hard section provided at a distal end of the flexible pipe, which is applied when a site to be repaired has occurred on an outer skin configuring an outer surface of the bending section according to use of the endoscope, comprises: a first step of removing a first string-winding section coupling the bending section and the distal end hard section, while removing a second string-winding section coupling the bending section and the soft section, and removing the outer skin where the site to be repaired has occurred from an outer skin of the bending section; a second step of attaching a new outer skin to an outer surface of the bending section and causing a distal end of the new outer skin to overlap with the distal end hard section, and causing a proximal end of the new outer skin to overlap with the soft section; a third step of winding a first string on the distal end of the new outer skin and the distal end hard section and fixedly coupling the new outer skin and the distal end hard section to each other thereby configuring a first string-winding section, and winding a second string on a proximal end of the new outer skin and the soft section and fixedly coupling the new outer skin and the soft section, thereby configuring a second string-winding section; a fourth step of applying adhesives to the first string-winding section and the second string-winding section, respectively; a fifth step of fitting thermally shrinkable tubes on the first string-winding section and the second string-winding section through clearances, respectively; and a sixth step of heating and shrinking the thermally shrinkable tube to tightly attach the thermally shrinkable tubes on the first string-winding section and the second string-winding section via the adhesives, respectively.

By replacing only an outer skin of the bending section where a site to be repaired has occurred by a new outer skin, fixedly coupling a distal end and a proximal end of the new outer skin by utilizing a string winding portion, and applying adhesive to the string-winding portion, and fitting a thermally-shrinkable tube thereon to conduct heating shrinkage of the tube in this manner, repair can be performed relatively easily in a short time so that workability can be improved and an endoscope that can withstand long-term use without impairing mechanical strength of a flexible pipe can be obtained.

According to the present invention, such an effect can be achieved that a site to be repaired occurring in an outer skin of a flexible pipe according to use of an endoscope can be repaired relatively easily in a short time so that improvement in workability can be achieved, and the endoscope can withstand long-term use without the mechanical strength of a flexible pipe being impaired.

### Brief Description of Drawings

FIG. 1 is a perspective view of an appearance of an endoscope according to a first embodiment of the present invention;
FIG. 2 is a vertical sectional view of a portion of a flexible pipe of the endoscope according to the first embodiment;
FIG. 3 is a vertical sectional view of a portion of the flexible pipe of the endoscope according to the first embodiment where a site to be repaired has occurred;
FIG. 4 is a vertical sectional view of a main portion for explaining a work for repairing a site to be repaired of the endoscope according to the first embodiment;
FIG. 5 is a vertical sectional view of a portion of the flexible pipe in a state that repair of the endoscope according to the first embodiment has been completed;
FIG. 6 is a vertical sectional view of a main portion for explaining a work for repairing a site to be repaired according to a repair method of an endoscope according to a second embodiment of the present invention;
FIG. 7A is a perspective view of a distal end portion of an insertion section of an endoscope; and
FIG. 7B is a vertical sectional view of an outer skin portion on a bending section that has been repaired according to a repair method of an endoscope.

### Best Mode for Carrying Out the Invention

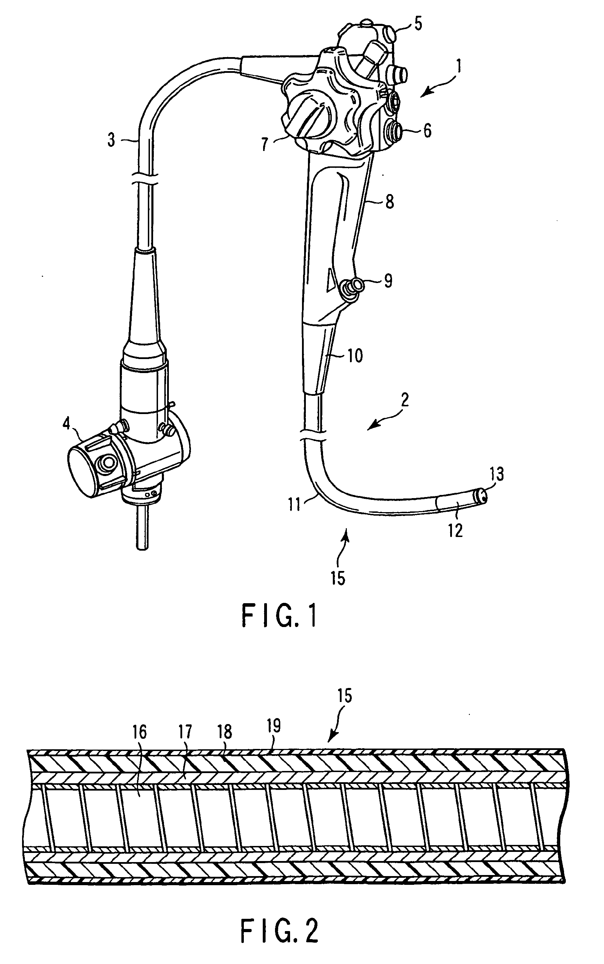

An endoscope according to an embodiment of the present invention will be explained with reference to the drawings. FIG. 1 is a perspective view of an endoscope of an electronic scope type and FIG. 2 is a partially sectional view of a flexible pipe 15.

As shown in FIG. 1, an endoscope includes an operation section 1 and an insertion section 2 provided at a distal end of this operation section 1. The operation section 1 is coupled with a proximal end portion of a universal cord 3. A scope connector 4 connected to a light source apparatus (not shown) is provided at a distal end of the universal cord 3.

The operation section 1 is provided with a plurality of switches 5. The plurality of switches 5 includes, for example, a switch for performing operation for recording a still image and causing the image to get still except for a video printer, a photometry switch for performing switching between averaging photometry and peak photometry, a switch for performing printout to the video printer, and the like.

The operation section 1 is provided with buttons 6 such as a suction button, and an air-supplying/water-supplying button. The suction button is configured such that suction is conducted from a distal end of the insertion section 2 when the suction button is pushed in. The air-supplying/water-supplying button is configured such that air is supplied from a distal end of the nozzle when a small hole at the center of the button is closed by a finger of an operator and water is supplied from the nozzle when the button is pushed in.

The operation section 1 is provided with knobs 7 such as a vertical angle knob, a horizontal angle knob, and a releasing knob. The vertical angle knob causes a bending section 12 configuring the insertion section 2 and described later to bend upwardly and downwardly. The horizontal angle knob causes the bending section 12 to bend leftward and rightward. The releasing knob fixes and unfixes the vertical angle knob and the horizontal angle knob.

A grasping section 8 is provided adjacent to the switches 5, the buttons 6 and the knobs 7. While an operator grasps the grasping section 8 by his/her one hand, he/she can operate the switches 5, the buttons 6, and the knobs 7 by his/her other hand. A forceps channel 9 in which a pair of forceps or the like is inserted is provided at a distal end of the grasping section 8.

The insertion section 2 comprises a soft section 11, the bending section 12, and a distal end hard section 13. A proximal end portion of the soft section 11 is protected by a breaking-preventing unit 10. The insertion section 2 is configured to be capable of bending along a body cavity shape in a state that it has been inserted into a body cavity of a patient. The bending section 12 is provided at a distal end of the soft section 11. The bending section 12 is bent and deformed according to operation of the knobs 7 on the operation section 1. The distal end hard section 13 is provided at a distal end of this bending section 12. The distal end hard section 13 includes the nozzle and lenses (an objective optical lens system)serving according to operation to the buttons 6 on the operation section 1, a forceps outlet also serving as a suction port, a light emitting end portion of a light guide, and the like.

Since both the soft section 11 and the bending section 12 have flexibility, they are collectively called "a flexible pipe 15". As shown in FIG. 2, this flexible pipe 15 comprises a flex (a helical pipe) 16, a blade (a mesh pipe) 17, and an outer skin 18. The blade 17 is provided on a surface of the flex 16. The outer skin 18 is provided so as to cover a surface of the blade 17. A surface of the outer skin 18 is covered with a topcoat layer 19 made from resin or the like.

The flex 16 is a pipe body with a flexibility formed in a hollow shape. A hollow portion of the flex 16 is inserted with various tubes (not shown) such as a channel tube, an optical fiber. Thermoplastic elastomer resin such as polyester, polyurethane, olefin, or styrene is used as material for the outer skin 18. For example, fluorinated material or polyurethane material is used as the topcoat layer 19.

Examination or procedure is performed using the endoscope thus configured. That is, an operator grasps the grasping section 8 on the operation section 1 to insert the insertion section 2 up to a desired site in a body cavity of a patient. The soft section 11 configuring the insertion section 2 is freely bent along the shape of the body cavity. The operator operates the operation section 1 to bend the bending section 12 when the distal end hard section 13 has reached the desired site. Thereby, the operator causes the distal end hard section 13 to face an affected area.

An image on the desired site is focused on an imaging device such as a CCD using an objective lens provided in the distal end hard section 13. Image data obtained by converting the image to electric signals by the imaging device is transmitted to an external video processor or the like via a signal line to be displayed on a screen of a monitor as an image. In a case of a fiber scope, an image obtained by the objective lens is taken in an eyepiece unit via an image guide. The operator inserts a corresponding surgical instrument into the forceps channel 9. At this time, the operator performs examination or procedure of the affected area based upon image data while confirming the affected area with an eye through observation of the endoscope.

The endoscope after used for performing required examination/procedure is cleaned, disinfected, and sterilized in order to avoid infection. The endoscope that has been subjected to this sequence of processes is stored in a clean atmosphere or under conditions close to a clean atmosphere and is provided in the next use.

When the use and management described above are repeated for a prolonged period, the outer skin 18 configuring an outer surface of the flexible pipe 15 is worn, which may result in a defect. Such a defect portion is called "a site to be repaired S", and such repair as described below will be performed.

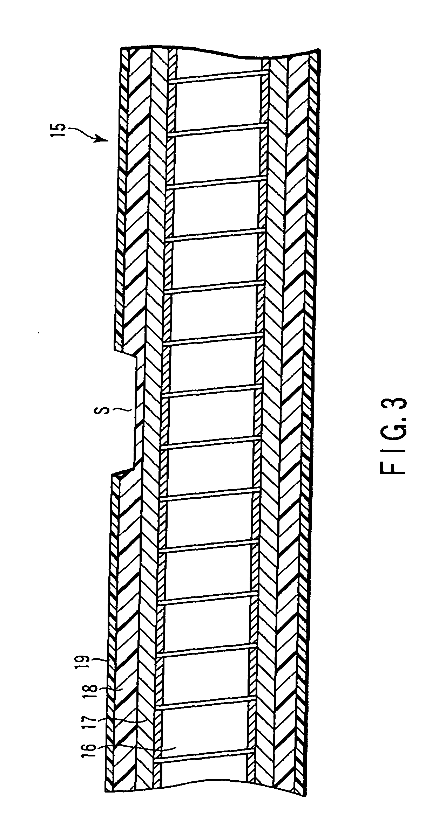

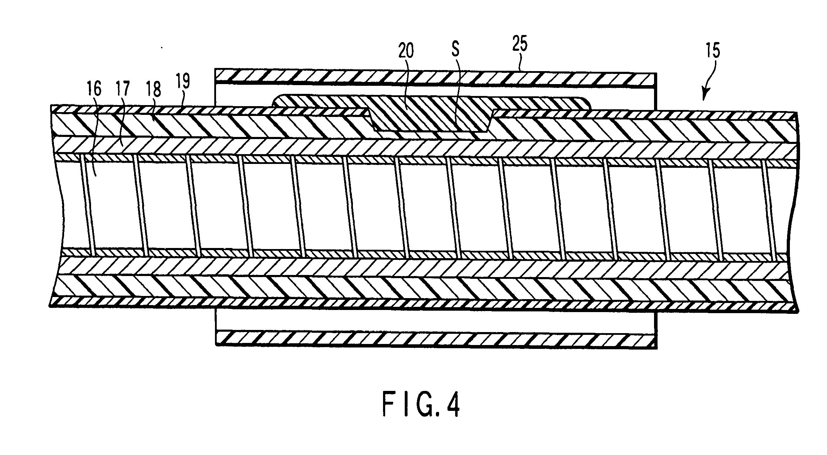

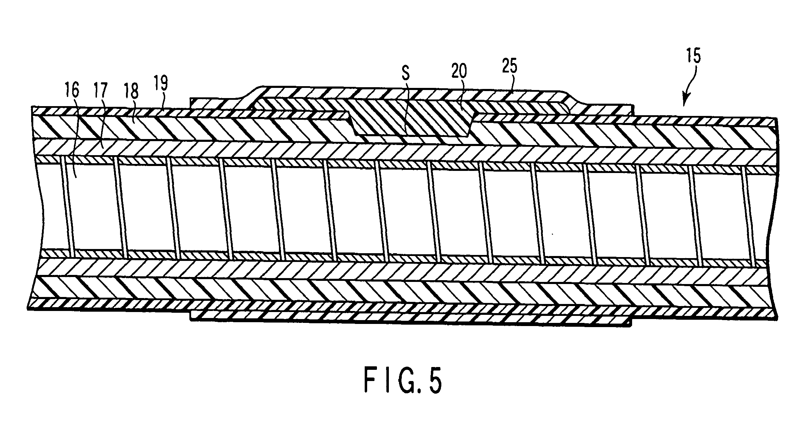

FIG. 3 is a vertical sectional view of a portion of the flexible pipe 15 where a site to be repaired S has occurred according to use, FIG. 4 is a vertical sectional view of a main portion for explaining a repair process to the site to be repaired S, and FIG. 5 is a vertical sectional view of a portion of the flexible pipe 15 whose repair has been completed.

Specifically, the flex 16 which is the helical pipe and the blade 17 which is the mesh pipe that configure the flexible pipe 15 have flexibility by themselves, but they have such stiffness that they are not damaged easily and are covered with the outer skin 18. Accordingly, the flex 16 and the blade 17 are not only reduced in wear but also they are reduced in occurrence of direct defects. On the other hand, since the outer skin 18 provided on the outer surface of the blade 17 is made from, for example, a thermoplastic elastomer resin, which is a relatively soft material, a defect easily occurs on the outer skin 18. Since the topcoat layer 19 is a thin film, a defect may occur on the outer skin 18 further easily.

Therefore, when the endoscope is used for a prolonged period, as shown in FIG. 3, recessed defect portions occur at a portion of the outer skin 18 and at a portion of the topcoat layer 19 due to wearing. The recessed defect portions are the portions to be repaired S. One example of a repair method for repairing such a site to be repaired S of the outer skin 18 is as follows.

As shown in FIG. 4, a first step of applying adhesive 20 to a site to be repaired S on an outer skin 18 is first performed. For example, epoxy-based two-liquid type thermosetting adhesive is used as the adhesive 20. It is preferable that the adhesive 20 is not only concentrically applied to only the site to be repaired S but also it is applied to a peripheral area surrounding the site to be repaired S.

After adhesive 20 is applied to the site to be repaired S and the peripheral area thereof, a second step of fitting a thermally shrinkable tube 25 on the flexible pipe 15 such that the tube 25 faces the adhesive 20 and gaps are present between the same and the flexible pipe 15 and between the same and the adhesive 20 is performed.

A thickness of the thermally shrinkable tube 25 is not limited to a specific one, and since the thermally shrinkable tube 25 is configured as a portion of the flexible pipe 15 after the repair has been completed, it may be required to have an essential thickness as far as the thickness does not cause any trouble in use of the endoscope. The thermally shrinkable tube 25 is made of a resin molded product mainly formed from, for example, polyvinyl chloride, polyethylene terephtalate resin, or ionomer resin.

As a third step, the thermally shrinkable tube 25 is heated to be thermally shrunk. As shown in FIG. 5, the thermally shrinkable tube 25 is tightly bonded to the outer surface of the flexible pipe 15 via the adhesive 20 applied to the outer surface of the flexible pipe 15 according to heating shrinkage thereof.

The thermally shrinkable tube 25 is fixed on the outer surface of the flexible pipe 15 in this manner, so that lowering of mechanical strength of the flexible pipe 15 due to the defect portion which is the site to be repaired S is prevented. The defect portion is a recessed portion and if the recessed defect portion is left as it is, it easily changes to a sharp recessed portion so that smoothness of the flexible pipe 15 is impaired, which results in large influence on a body cavity surface. Since the defect portion is covered with the thermally shrinkable tube 25 with the smooth outer surface, smoothness on the surface of the flexible pipe 15 can be maintained.

Incidentally, it is inevitable that the adhesive 20 applied to the site to be repaired S at the second step is dried and adhesion force is lost to some extent at a starting time of the third step. However, since the thermally shrinkable tube 25 is heated and shrunk to come in close contact with the adhesive 20 in the third step so that heat is transferred to the adhesive 20, the adhesive 20 melts and the adhesion force recovers. As a result, the thermally shrinkable tube 25 can be firmly adhered and fixed to the surface of the flexble pipe 15.

As explained above, in the repair method according to the present embodiment, after the adhesive 20 is applied to the site to be repaired S, the thermally shrinkable tube 25 is fitted on the site to be repaired S, and repair of the site to be repaired S is terminated by only heating and shrinking the thermally shrinkable tube 25. Accordingly, repair can be conducted rapidly with a relatively problem-free simple work. As a result, the repair time is short, the endoscope can be provided for re-use in a short time, and the mechanical strength of the flexible pipe 15 is prevented from lowering so that the flexible pipe 15 can withstand long-term use.

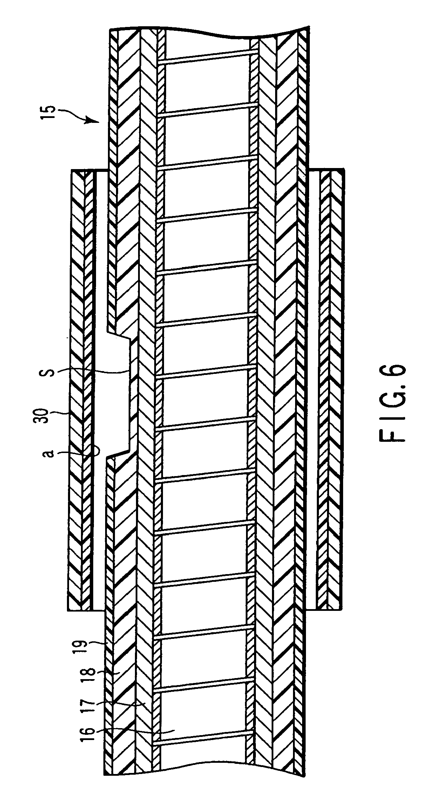

FIG. 6 is a vertical sectional view of a main portion for explaining a work for repairing a site to be repaired that is performed by a repair method of an endoscope according to a second embodiment of the present invention. In a repair method of an endoscope according to the present embodiment, a thermally shrinkable tube 30 having an adhesive layer "a" on an inner peripheral face thereof is fitted on a site to be repaired S of an outer skin 18 and a peripheral area thereof with a clearance as a first step.

As the thermally shrinkable tube 30, for example, one disclosed in Jpn. Pat. Appln. KOKAI Publication No. 05-8335 is used. The thermally shrinkable tube 30 is a thermally shrinkable tube with a multilayered structure of a foamed body. The thermally shrinkable tube 30 can easily tightly contact/cover a surface of an article such as a metal pipe or a rod, and since it gives adiabaticity, insulation property, and anticorrosion/waterproof property to the article, it is suitable for protection.

The thermally shrinkable tube disclosed in the Publication is characterized in that it includes at least one foamed body layer made from thermoplastic polymer and at least one non-foamed body layer made from thermoplastic polymer, and it further includes a hot-melt type adhesive layer as an innermost layer.

Even if the density of the foamed layer that is foamed by heating lowers, since this thermally shrinkable tube has the non-foamed layer, the mechanical strength of the tube can be maintained. Since the hot-melt type adhesive layer melts to adhere to a surface of an article, the thermally shrinkable tube configures a tube with high adhesiveness to a surface of an article.

As the thermally shrinkable tube 30, for example, one disclosed in Jpn. Pat. Appln. KOKAI Publication No.06-55636 may be used. The tube comprises a resin molded product including ionomer resin as a main component and an adhesive layer formed on an inner face of the resin molded product. Utilizing the resin molded product including ionomer resin as the main component is advantageous in that high transparency is obtained and such a problem as shrinking of the product in a longitudinal direction thereof does not occur even if thermally shrinking work is conducted.

As the thermally shrinkable tube 30, for example, one disclosed in Jpn. Pat. Appln. KOKAI Publication No. 2001-260224 may be used. The thermally shrinkable tube disclosed in the Publication is a tube provided with an inner face thereof with an adhesive layer of heat-melting type. Regarding this tube, after the tube is thermally bonded to an outer surface of a steel pipe by heating the tube to thermally shrink the same, the thermally adhesive layer covering the steel pipe is remelted by further induction-heating the steel pipe. Thereby, the adhesive spreads over a face to be bonded in every corner, and evenness of a thermally shrinking amount of the tube is achieved, which is advantageous in that adhesion force is increased largely.

Though either one of the thermally shrinkable tubes 30 may be used, one having an inner diameter slightly larger than a diameter of the flexible pipe 15 is selected. That is, though the thermally shrinkable tube 30 is provided on its inner face with the adhesive layer "a", it can have such a diameter that the flexible pipe 15 can be easily inserted into the tube before the tube is heated and shrunk and the tube comes in close contact with the outer surface of the flexible pipe 15 after it is heated and shrunk.

A length of the thermally shrinkable tube 30 in an axial direction thereof is set so as to be capable of covering at least a site to be repaired S that has occurred on an outer skin 18 sufficiently. It is preferable that the tube has such a length that it can cover the site to be repaired S and a peripheral portion thereof. A thickness of the thermally shrinkable tube 30 is not limited to a specific one, but it may be an essential thickness for actual use as a portion of the flexible pipe 15.

Next, as a second step, the thermally shrinkable tube 30 is heated and shrunk. The thermally shrinkable tube 30 comes in close contact with an outer surface of the flexible pipe 15, and heat received by the thermally shrinkable tube 30 at this time transfers to the adhesive layer "a" on the inner peripheral face to melt the adhesive.

The melted adhesive of the adhesive layer "a" flows to the site to be repaired S and the peripheral portion. Therefore, the thermally flexible tube 30 is brought in close contact with and fixed on the outer surface of the flexible pipe 15 by the adhesive flowing to the peripheral portion. Eventually, repair is completed in the same manner as the repair method previously explained.

As described above, in the repair method of the endoscope according to the present embodiment, repair of a site to be repaired S which has occurred on a flexible pipe 15 is completed by only heating and shrinking the thermally shrinkable tube 30 having the adhesive layer "a" on an inner face thereof after fitting it on the site to be repaired S. That is, the repair can be rapidly conducted by a relatively problem-free and simple work. Therefore, while a repair time is reduced and the endoscope can be provided for re-use thereof in a short amount of time, lowering of mechanical strength of the flexible pipe 15 is prevented so that the endoscope can withstand long-term use.

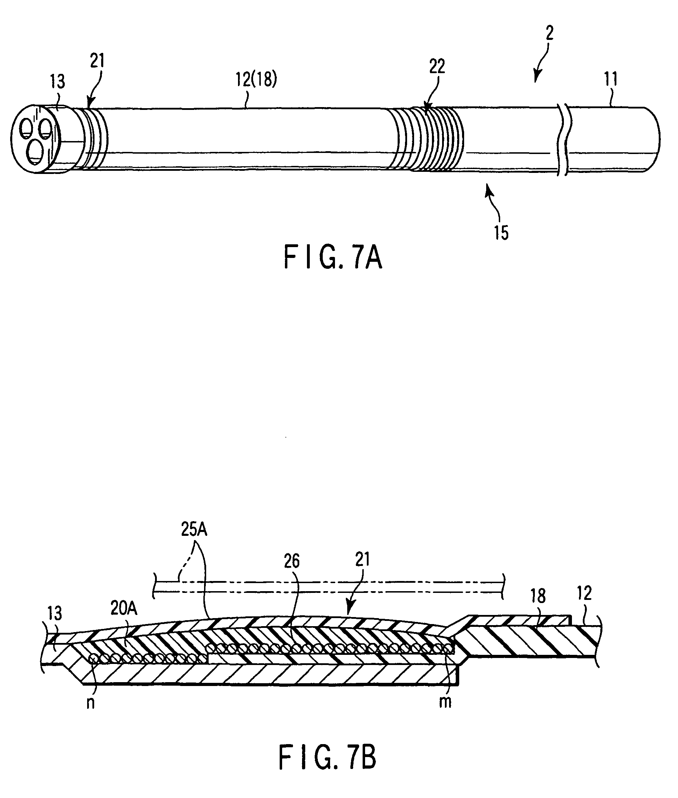

Next, a repair method of an endoscope when a site to be repaired S has occurred on an outer skin 18 configuring an outer surface of a bending section 12 according to use of an endoscope will be explained with reference to FIGS. 7A and 7B. FIG. 7A is an enlarged perspective view of a distal end portion of an insertion section of an endoscope and FIG. 7B is a view for explaining a repair method of an endoscope.

The bending section 12 is integrally coupled with a distal end hard section 13 at a distal end thereof and it is integrally couple with a soft section 11 at a proximal end thereof. Specifically, for manufacture of the endoscope insertion section 2, an outer peripheral face of a coupling portion of the bending section 12 and the distal end hard section 13 is wound by a string. Thereby, a first string-winding section 21 fixedly coupling both the sections is provided. Similarly, an outer peripheral face of a coupling portion of the bending section 12 and the soft section 11 is wound by a string. Thereby, a second string-winding section 22 fixedly coupling both the sections is provided.

After the first and second string-winding sections 21 and 22 have been provided, the insertion section 2 is automatically fed to an adhesive applying apparatus. Thereby, adhesive is automatically applied to an outer peripheral faces of the first and second string-winding sections 21 and 22. Therefore, surfaces of the first and second string-winding sections 21 and 22 maintain smoothness in a state that the endoscope has been finished as a product.

As the first step, at a repair time of the bending section 12 of the endoscope, the first string-winding section 21 and the second string-winding section 22 are removed. Thereby, the outer skin 18 where the site to be repaired S has occurred is removed from the outer surface of the bending section 12. Specifically, applied and solidified adhesive to the first and second string-winding sections 21 and 22 is first removed. After the adhesive has been completely removed, strings wound on the respective string-winding sections 21 and 22 are unwound to be removed. After this state is reached, the outer skin 18 where the site to be repaired S has occurred is removed from the bending section 12.

As a second step, a new outer skin 18 is attached on an outer surface of the bending section 12. A distal end of the outer skin 18 is then caused to overlap with the distal end hard section 13. Similarly, a proximal end of the outer skin 18 is caused to overlap with the soft section 11 to be held.

FIG. 7B is a sectional view of the first string-winding section 21 coupling the bending section 12 and the distal end hard section 13 with each other. The first string-winding section 21 will be explained below. Since exactly the same configuration and the same repair method are applied to the second string-winding section 22 coupling the bending section 12 and the soft section 11, detail explanation about the second string-winding section 22 will be omitted by substituting the explanation about the first string-winding section 21.

As a third step, a string 26 is wound on an overlapping portion of the distal end of the new outer skin 18 and the distal end hard section 13. That is, the distal end of the new outer skin 18 which has been caused to overlap is caused to overlap with a surface of the distal end hard section 13, and the string 26 are wound on the overlapped portions without clearance to be fixed. Thereby, a new first string-winding section 21 is formed.

Winding of the string 26 is started from a position of a winding-starting portion "m" at a proximal end portion of a small-diameter portion forming a distal end of the new outer skin 18 and the string 26 reaches a distal end of the small-diameter portion. The string 26 is wound over the proximal end portion of the distal end hard section 13 as it is, and winding thereof is terminated at a winding-terminating portion "n" that is a stepped portion of the distal end hard section 13. End portions of the string 26 are inserted below the string 26 which has been wound between the winding-staring portion "m" and the winding-terminating portion "n" and has been fixed.

As a fourth step, adhesive 20A is applied to an outer peripheral face of the first string-winding section 21 in the state that the string 26 has been wound. As the adhesive 20A, for example, epoxy-based two-liquid type thermosetting adhesive may be used. Thereby, water-tightness at the first string-winding section 21 that is a binding site between the distal end hard section 13 and the binding section 12 is ensured.

As a fifth step, a thermally shrinkable tube 25A is fitted on the first string-winding section 21 with a clearance therebetween. The thermally shrinkable tube 25A has an inner diameter larger than the diameters of the distal end hard section 13 and the bending section 12 to some extent. The thermally shrinkable tube 25A is only required to allow insertion of the distal end hard section 13 and the bending section 12 therein before heated and shrunk and shrink until it comes in close contact with the bending section 12 after heated and shrunk.

A length of the thermally shrinkable tube 25A in an axial direction thereof may be set to such a size that the tube can cover at least the first string-winding section 21 sufficiently. It is further preferable that the length of the thermally shrinkable tube 25A in the axial direction is set such that the tube can cover the first string-winding section 21 and a peripheral site thereof.

A thickness of the thermally shrinkable tube 25A is not limited to a specific one, but since the thermally shrinkable tube 25A is used as a portion of the flexible pipe 15 after repair, the thickness is set to an essential one. In view of such a function that a surface of the thermally shrinkable tube 25A is inserted into a body cavity of a human, material having smoothness must be selected for the thermally shrinkable tube 25A. The thermally shrinkable tube 25A comprises a resin-molded product mainly formed from, for example, polyvinyl chloride, polyethylene terephtalate resin, or ionomer resin.

As a sixth step, the thermally shrinkable tube 25A is tightly attached to the first string-winding section 21 by heating the thermally shrinkable tube 25A to shrink the same. According to the tight attachment of the thermally shrinkable tube 25A to the first string-winding section 21, heat absorbed by the thermally shrinkable tube 25A transfers to the first string-winding section 21. Thereby, the applied adhesive 20A melts so that the thermally shrinkable tube 25A tightly fixes to an outer surface of the bending section 12 of the thermally shrinkable tube 25A to be fixed thereon. The repair is completed at this state.

By covering the first string-winding section 21 with the thermally shrinkable tube 25A, it is made possible to conceal a surface of the first string-winding section 21 that is difficult to form smoothly in a state that the adhesive 20A has been applied to the surface. That is, though the adhesive 20A is applied to the first string-winding section 21 at the fourth step, since the applying work is conducted manually, smoothness on the first string-winding section 21 is inferior to that obtained by automatic application of adhesive performed using an adhesive-applying apparatus at a manufacturing time of an endoscope. Therefore, by covering the first string-winding section 21 applied with the adhesive 20A with the thermally shrinkable tube 25A at the sixth step, smoothness to be originally possessed by the flexible pipe 15 can be maintained, and any trouble does not occur in examination/procedure using an endoscope.

Some degree of water-tightness is maintained by applying the adhesive 20A to the first string-winding section 21 at the fourth step, but the water-tightness can be further enhanced, since the first string-winding section 21 applied with the adhesive 20A is covered with the thermally shrinkable tube 24A at the sixth step.

Incidentally, as described above, the step of coupling the replaced new outer skin 18 and the soft section 11 is performed in exactly the same manner as the abovementioned coupling step. Accordingly, application of all explanation is made possible by substituting the soft section 11 for the distal end hard section 13 and substituting the second string-winding section 22 for the first string-winding section 21 at the abovementioned steps.

The following effect can be achieved in the abovementioned method. That is, the effect can be achieved by removing the outer skin 18 of the bending section 12 where a site to be repaired S has occurred according to use of an endoscope to perform application of a new outer skin 18, fixedly coupling the new outer skin 18 with the distal end hard section 13 via the first string-winding section 21, fixedly coupling the new outer skin 18 with the soft section 11 via the second string-winding section 22, and shrinking the thermally shrinkable tubes 25A after applying the adhesive 20A to the first and second string-winding sections 21 and 22. Thereby, the outer skin 18 at the bending section 12 comprises a new one, and repair can be performed rapidly with a relatively problem-free simple work. Therefore, the repair time is short, the endoscope can be provided for re-use in a short time, and the mechanical strength of the bending section 12 is prevented from lowering so that the endoscope can withstand long-term use.

The present invention is not limited to the abovementioned embodiment as it is, and it may be embodied in an implementation stage by modifying constituent elements without departing from the gist of the present invention. Various inventions can be configured according to proper combinations of a plurality of constituent elements disclosed in the abovementioned embodiment.

## Claims

1. A method for repairing an endoscope comprising a flexible pipe (15) configured to be inserted into a body cavity, wherein said flexible pipe (15) comprises a flex (16), a blade (17) provided on the flex (16), an outer skin (18) covering the blade (17) and a top coat layer (19) made from resin covering the outer skin (18), a recessed defect portion, which is to be repaired, formed at a portion of the outer skin (18) and at a portion of the top coat layer (19), and a peripheral area on an outer surface of the flexible pipe (15) surrounding the recessed portion, **characterized by** the steps of:
concentrically applying an adhesive (20) to the recessed portion and the peripheral area;
fitting a thermally shrinkable tube (25) on the recessed portion and the peripheral area; and
heating the thermally shrinkable tube (25) to thermally-shrink the thermally-shrinkable tube (25), and tightly attach the thermally shrinkable tube (25) to the outer surface of the flexible pipe (15) via the adhesive (20).

2. The method according to claim 1, **characterized in that**
the heating includes transferring heat to the adhesive (20) via the thermally shrinkable tube (25) to melt the adhesive (20) and recover adhesive effect.

3. A method for repairing an endoscope comprising a flexible pipe (15) configured to be inserted into a body cavity, wherein said flexible pipe (15) comprises a flex (16), a blade (17) provided on the flex (16), an outer skin (18) covering the blade (17) and a top coat layer (19) made from resin covering the outer skin (18), a recessed defect portion, which is to be repaired, formed at a portion of the outer skin (18) and at a portion of the topcoat layer (19), and a peripheral area on an outer surface of the flexible pipe (15) surrounding the recessed portion, **characterized by** the steps of:
providing a thermally shrinkable tube (30) including an adhesive layer (a) on an inner surface of the thermally shrinkable tube (30);
fitting the thermally shrinkable tube (30) on the recessed portion formed during use of the endoscope and the peripheral area; and
heating the thermally shrinkable tube (30) to thermally-shrink the thermally shrinkable tube (30), transfer heat to the adhesive layer (a) via the thermally shrinkable tube (30) to melt the adhesive layer (a) to flow an adhesive of the adhesive layer (a) to the recessed portion and the peripheral area, and tightly attach the thermally shrinkable tube (30) to the outer surface of the flexible pipe (15) via the adhesive.

## Patentansprüche

1. Verfahren zum Reparieren eines Endoskops mit einem flexiblen Rohr (15), das zum Einführen eine Körperhöhle ausgebildet ist, wobei das flexible Rohr (15) ein Anschlussrohr (16), eine an dem Anschlussrohr vorgesehene Auflage (17), eine die Auflage (17) bedeckende Außenhaut (18) und eine obere Mantelschicht (19) aus Harz, die die Außenhaut (18) überdeckt, einen vertieften defekten Abschnitt, der zu reparieren ist und an einem Abschnitt der Außenhaut (18) und einem Abschnitt der oberen Mantelschicht (19) gebildet ist, und einen Umfangsbereich an einer Außenfläche des flexiblen Rohrs (15) umfasst, der den vertieften Abschnitt umgibt, **gekennzeichnet durch** die Schritte:
Konzentrisches Auftragen eines Klebstoffs (20) auf den vertieften Abschnitt und den Umfangsbereich;
Anbringen eines thermisch schrumpfbaren Rohrs an dem vertieften Abschnitt und dem Umfangsbereich; und
Erwärmen des thermisch schrumpfbaren Rohrs (25), um das thermisch schrumpfbare Rohr (25) thermisch zu schrumpfen und das thermisch schrumpfbare Rohr (25) mittels des Klebstoffs (20) fest auf der Außenfläche des flexiblen Rohres anzubringen.

2. Verfahren nach Anspruch 1, **dadurch gekennzeichnet, dass** das Erwärmen das Übertragen der Wärme auf den Klebstoff (20) über das thermisch schrumpfbare Rohr (25) umfasst, um den Klebstoff zu schmelzen und den Hafteffekt wieder zu gewinnen.

3. Verfahren zum Reparieren eines Endoskops mit einem zum Einführen in eine Körperhöhle ausgebildeten flexiblen Rohr (15), wobei das flexible Rohr (15) ein Anschlussrohr (16), eine an dem Anschlussrohr (16) anzubringende Auflage (17), eine die Auflage (17) bedeckende Außenhaut (18) und eine obere Mantelschicht (19) aus Harz, die die Außenhaut (18) bedeckt, einen vertieften defekten Abschnitt, der zu reparieren ist und an einem Abschnitt der Außenhaut (18) und einem Abschnitt der oberen Mantelschicht (19) gebildet ist, und einen Umfangsbereich an einer Außenfläche des flexiblen Rohrs (15) umfasst, der den vertieften Abschnitt umgibt, **gekennzeichnet durch** die Schritte:
Bereitstellen eines thermisch schrumpfbaren Rohrs (30), das eine Haftschicht (a) an einer Innenfläche des thermisch schrumpfbaren Rohrs (30) umfasst;
Anbringen des thermisch schrumpfbaren Rohrs (30) an dem während der Verwendung des Endoskops gebildeten vertieften Abschnitt und dem Umfangsbereich; und
Erwärmen des thermisch schrumpfbaren Rohrs (30) zum thermischen Schrumpfen des thermisch schrumpfbaren Rohrs (30), Übertragen der Wärme auf die Haftschicht (a) über das thermisch schrumpfbare Rohr (30) zum Schmelzen der Haftschicht (a), um einen Fluss des Klebstoffs der Haftschicht (a) zu dem vertieften Abschnitt und dem Umfangsbereich zu erreichen, und festes Anbringen des thermisch schrumpfbare Rohr (30) an der Außenfläche des flexiblen Rohrs (15) mittels des Klebstoffs.

## Revendications

1. Procédé de réparation d'un endoscope comprenant un tuyau flexible (15) configuré pour être inséré dans une cavité corporelle, dans lequel ledit tuyau flexible (15) comprend un flexible (16), une lame (17) prévue sur le flexible (16), une peau extérieure (18) couvrant la lame (17) et une couche supérieure (19) constituée d'une résine couvrant la peau extérieure (18), une partie défectueuse en renfoncement, qui doit être réparée, formée au niveau d'une partie de la peau extérieure (18) et au niveau d'une partie de la couche supérieure (19), et d'une zone périphérique sur une surface extérieure du tuyau flexible (15) entourant la partie en renfoncement, **caractérisé par** les étapes de :
application concentrique d'une adhésif (20) à la partie en renfoncement et la zone périphérique ;
mise en place d'un tube thermorétractable (25) sur la partie en renfoncement et la zone périphérique ; et
chauffage du tube thermorétractable (25) pour thermorétracter le tube thermorétractable (25), et fixer étroitement le tube thermorétractable (25) à la surface extérieure du tuyau flexible (15) par l'intermédiaire de l'adhésif (20).

2. Procédé selon la revendication 1, **caractérisé en ce que**
le chauffage inclut un transfert de chaleur à l'adhésif (20) par l'intermédiaire du tube thermorétractable (25) pour faire fondre l'adhésif (20) et récupérer l'effet adhésif.

3. Procédé de réparation d'un endoscope comprenant un tuyau flexible (15) configuré pour être inséré dans une cavité corporelle, dans lequel ledit tuyau flexible (15) comprend un flexible (16), une lame (17) prévue sur le flexible (16), une peau extérieure (18) couvrant la lame (17) et une couche supérieure (19) constituée d'une résine couvrant la peau extérieure (18), une partie défectueuse en renfoncement, qui doit être réparée, formée au niveau d'une partie de la peau extérieure (18) et au niveau d'une partie de la couche supérieure (19), et d'une zone périphérique sur une surface extérieure du tuyau flexible (15) entourant la partie en renfoncement, **caractérisé par** les étapes de :
prévision d'un tube thermorétractable (30) incluant une couche adhésive (a) sur une surface intérieure du tube thermorétractable (30) ;
mise en place du tube thermorétractable (30) sur la partie en renfoncement formée pendant l'utilisation de l'endoscope et la zone périphérique ; et
chauffage du tube thermorétractable (30) pour thermorétracter le tube thermorétractable (30), transférer une chaleur à la couche adhésive (a) par l'intermédiaire du tube thermorétractable (30) pour faire fondre la couche adhésive (a) pour faire s'écouler un adhésif de la couche adhésive (a) jusqu'à la partie en renfoncement et la zone périphérique, et fixer étroitement le tube thermorétractable (30) à la surface extérieure du tuyau flexible (15) par l'intermédiaire de l'adhésif.
